# FASCICULE DE BREVET EUROPEEN

(11) **EP 1 609 456 B1**
(45) Date de publication et mention de la délivrance du brevet: **28.10.2009**
(21) Numéro de dépôt: 05291138.5
(22) Date de dépôt: 27.05.2005
(51) Int. Cl.: A61Q 5/06, A61K 8/49

(54) **Utilisation de composés azoïques polycationiques en teinture des fibres kératiniques**
Verwendung von polykationischen Azofarbstoffen zur Färbung von Keratinfasern
Use of polycationic azo compounds for dyeing keratinous fibres

(30) Priorité: 28.05.2004 FR 0405805
(43) Date de publication de la demande: 28.12.2005
(73) Titulaire: L'ORÉAL, 75008 Paris (FR)
(72) Inventeur: Lagrange, Alain, 77700 Coupvray (FR); David, Hervé, 94340 Joinville le Point (FR); Greaves, Andrew, 77144 Montevrain (FR)
(74) Mandataire: Casalonga, Axel

(56) Documents cités:
- WO-A-02/100365
- WO-A-03/029359
- US-A- 5 708 151
- TECHNIQUES DE L'INGENIEUR COLORANTS TEXTLES, 1988,

## Description

La présente demande a pour objet l'utilisation de composés azoïques polycationiques, à titre de colorants directs, dans des compositions tinctoriales pour la teinture des fibres kératiniques, et en particulier les cheveux humains. Elle vise également des compositions tinctoriales à base de ces colorants, ainsi que l'utilisation de ces compositions pour la teinture des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho-ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés. Ces colorants, insolubles dans le milieu de teinture, sont piégés à l'intérieur du cheveu.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques par une coloration directe ou semi-permanente. Le procédé classiquement utilisé en coloration directe consiste à appliquer sur les fibres kératiniques des colorants directs qui sont des molécules colorées et colorantes ayant une affinité pour les fibres, à laisser poser pour permettre aux molécules colorées de pénétrer, par diffusion, à l'intérieur du cheveu, puis à rincer les fibres.

Contrairement aux compositions de teinture par oxydation, les compositions de teinture directes ou semi-permanentes peuvent avantageusement être mises en oeuvre sans la présence obligatoire d'un agent oxydant. Ces teintures peuvent être effectuées de manière répétée sans dégrader la fibre kératinique.

Il est connu par exemple d'utiliser des colorants directs nitrés benzéniques, anthraquinoniques, nitropyridiniques, azoïques, xanthéniques, acridiniques, aziniques ou triarylméthaniques.

Document WO 02100365 décrit des composés dicationiques comprenant deux groupes à fonction colorante de type arylazoimidazolium avec un bras de liaison non ionique ainsi que leur utilisation comme colorant direct.

Il en résulte des colorations souvent chromatiques qui sont cependant temporaires ou semi-permanentes à cause de la nature des liaisons entre les colorants directs et la fibre kératinique. Ces interactions font que la désorption des colorants de la surface et/ou du coeur de la fibre se fait facilement. Les colorations présentent généralement une faible puissance tinctoriale et une mauvaise tenue aux lavages ou à la transpiration. Ces colorants directs sont en outre généralement sensibles à la lumière car la résistance du chromophore vis-à-vis des attaques photochimiques est souvent faible, ce qui conduit à un affadissement de la coloration des cheveux dans le temps.

Par ailleurs, les colorants directs classiques ne sont pas toujours complètement inoffensifs, c'est pourquoi en cosmétique capillaire, on recherche des molécules colorantes de ce type toujours plus performantes en terme d'innocuité.

Il existe un réel besoin de disposer de compositions de teinture directe améliorées en terme de tenue aux shampooings et de montée du colorant.

De manière surprenante et avantageuse, la Demanderesse vient de découvrir que l'utilisation de composés azoïques polycationiques particuliers dans des compositions pour la teinture des fibres kératiniques, en particulier des fibres kératiniques humaines telles que les cheveux, permettait d'obtenir des compositions tinctoriales qui présentent ces améliorations.

Outre leur avantage en terme d'innocuité, les compositions selon la présente demande permettent l'obtention de teintures résistantes aux agents extérieurs (tels que le soleil, les intempéries), ainsi qu'aux shampooings et à la transpiration, et permettent d'obtenir des reflets intenses et tenaces sur les fibres.

En outre, ces compositions présentent une montée améliorée, une sélectivité réduite, ainsi qu'un bon profil toxicologique, la sélectivité étant la différence de montée entre les différentes parties d'un cheveu ou d'une chevelure.

La présente invention a ainsi pour objet l'utilisation à titre de colorant direct dans des compositions tinctoriales pour fibres kératiniques, en particulier les fibres kératiniques humaines telles que les cheveux, ou pour la fabrication de telles compositions, d'un composé de formule A-L-B, dans laquelle A et B désignent un groupe à fonction colorante de type arylazoimidazolium, et L désigne un bras de liaison comprenant au moins un groupe cationique C.

L'invention a également pour objet une composition tinctoriale pour la teinture des fibres kératiniques, en particulier des fibres kératiniques humaines telles que les cheveux, comprenant dans un milieu approprié pour la teinture au moins un composé de formule A-L-B, tel que décrit ci-dessus, et au moins une base d'oxydation et/ou au moins un colorant direct additionnel.

Un autre objet de l'invention porte sur un procédé de teinture des fibres kératiniques, en particulier des fibres kératiniques humaines telles que les cheveux mettant en oeuvre les composés de formule A-L-B.

L'invention a également pour objet l'utilisation de la composition de la présente invention sur les fibres kératiniques, en particulier les fibres kératiniques humaines telles que les cheveux pour obtenir des teintures présentant une bonne résistance aux agents extérieurs et aux shampooings.

D'autres caractéristiques, aspects, objets et avantages de l'invention apparaîtront encore plus clairement à la lecture de la description et des exemples qui suivent.

A et B désignent, indépendamment, un groupe à fonction colorante porteur d'une charge cationique et de type arylazoimidazolium.

Plus particulièrement, le composé de formule A-L-B comprend deux groupes à fonction colorante (dichromophore).

Le groupe L représente un « bras de liaison », qui relie les groupes A et B. On entend par « bras de liaison » une chaîne hydrocarbonée, linéaire ou ramifiée, saturée ou insaturée, pouvant être terminée ou interrompue par un ou plusieurs groupements carbonyle et/ou par un ou plusieurs hétéroatomes, tel que par exemple O, N, ou Si, ou une chaîne comprenant un ou plusieurs cycles aromatiques ou un ou plusieurs hétérocycles aromatiques ou saturés, au moins un groupe cationique C étant intercalé dans la chaîne. Le ou les groupes cationiques C peuvent être intercalés à n'importe quel endroit de la chaîne L, et notamment aux extrémités de la chaîne.

La liaison entre les groupes A et B et le groupe L peut se faire avec n'importe quel atome du groupe L.

Le bras de liaison est par exemple une chaîne hydrocarbonée en C₁-C₄₀, plus particulièrement en C₁-C₂₀, de préférence en C₁-C₈, linéaire ou ramifiée, saturée ou insaturée, un ou plusieurs des atomes de carbone de la chaîne pouvant être remplacés par un hétéroatome tel que le soufre, l'azote, l'oxygène ; un radical (hétéro)arylène ; un radical divalent téréphtalamide ; un radical divalent triazine, un radical -NH-CO-.

La chaîne constituant le bras de liaison peut être substituée par exemple par un radical hydroxy, alcoxy, notamment en C₁-C₆, amino, alkylamino, notamment en C₁-C₆, un halogène.

A titre d'exemple de bras de liaison, on peut également citer les radicaux alkylène (CₙH₂ₙ) comprenant de préférence de 1 à 6 atomes de carbone, par exemple méthylène, éthylène, propylène, etc ; les radicaux (hétéro)arylène par exemple phénylène ou naphtylène, phénanthrylène, triazinyle, pyrimidinyle, pyridinyle, pyridazinyle, quinoxalinyle, les radicaux Alkyle-aryle-Alkyle.

A titre de bras de liaison, on peut également citer les triazines décrites dans le WO03/029359, les alkylènes cités dans US 5 708 151, les Alkyle-aryle-Alkyle cités dans US 5 708 151.

De manière préférée, le bras de liaison L est une chaîne hydrocarbonée en C₁-C₂₀, de préférence en C₁-C₈, linéaire ou ramifiée.

Le groupe cationique C peut être choisi parmi les groupements cationiques aliphatiques ou hétérocycliques.

Parmi les groupements cationiques hétérocycliques, on peut citer les groupements imidazolium, pipéridinium, pyridinium, pyrazolium, triazolium, de préférence un groupement imidazolium.

Les groupements cationiques aliphatiques peuvent être des radicaux divalents du type -N⁺-R₁R₂-, R₁ et R₂ désignant indépendamment l'un de l'autre un radical alkyle en C₁-C₁₀, mono ou polyhydroxyalkyle en C₁-C₁₀.

Le groupe cationique C est de préférence choisi parmi les groupements cationiques aliphatiques et les groupements imidazolium, pyridinium ou pipéridinium.

Le composé de formule A-L-B est de préférence choisi parmi les composés de formule (I) suivante : 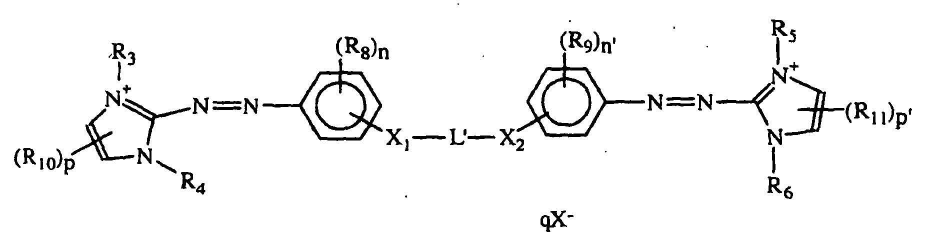 dans laquelle :
X₁, X₂ désignent indépendamment l'un de l'autre un cycle pipérazine substitué par un radical alkyle en C₁-C₈ ; un radical -O- ou -NR₇-, R₇ désignant un atome d'hydrogène, un radical alkyle en C₁-C₈, ou un radical hydroxyalkyle en C₁-C₈;
R₃ à R₆ désignent indépendamment les uns des autres un radical alkyle en C₁-C₈, ou un radical hydroxyalkyle en C₁-C₈;
R₈ et R₉ désignent un atome d'hydrogène, un radical alkyle en C₁-C₄ éventuellement substitué par un ou plusieurs radicaux choisis parmi les radicaux hydroxy, amino, (di)alkylamino en C₁-C₂ ; un radical carboxy ; un radical alcoxy en C₁-C₂ ; un radical amino ; un radical (di)alkylamino en C₁-C₂ ; un radical (poly)-hydroxyalkylamino en C₂-C₄ ;
R₁₀ et R₁₁ désignent, indépendamment l'un de l'autre, un atome d'hydrogène, un radical alkyle en C₁-C₄ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ; un radical phényle éventuellement substitué ; un radical carboxy ; un radical sulfonylamino ;
L' est un bras de liaison ; le groupement X₁-L'-X₂ (correspondant au groupement L) comprenant au moins un groupe cationique C ;
n et n' désignent un nombre entier allant de 0 à 4 ;
p et p' désignent un nombre entier allant de 0 à 2 ;
q désigne un nombre entier allant de 3 à 50, de préférence de 3 à 10, et encore plus préférentiellement de 3 à 5 ; q est choisi de préférence de façon à assurer la neutralité des charges du composé de formule (I) ;
X⁻ désigne un anion d'origine minérale ou organique ; X⁻ peut être choisi notamment parmi les ions halogénures, et en particulier chlorure et iodure, les ions sulfate ou hydrogénosulfate, les ions méthosulfate, tosylate, carbonate ou hydrogénocarbonate, phosphate, nitrate, citrate.

Le composé de formule A-L-B peut être par exemple choisi parmi les composés de formules suivantes : 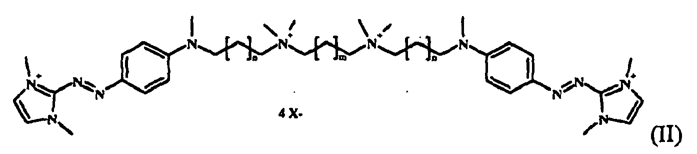 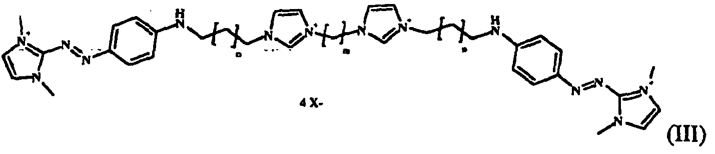 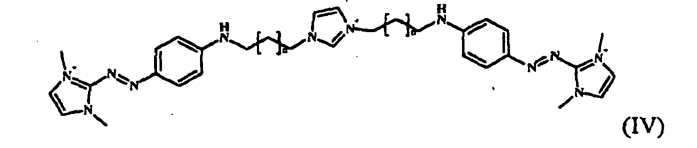 dans lesquelles :
X⁻ a la même signification que précédemment, et
m et n sont des entiers allant de 0 à 20 et de préférence de 0 à 8 ;
m et n étant tels que le bras de liaison comprend de 1 à 40, plus particulièrement de 1 à 20, de préférence de 1 à 8 atomes de carbone.

La composition selon la présente demande peut comprendre de 0,001 à 20%, de préférence de 0,01 à 10%, et de manière encore préférée 0,1 à 5% en poids de colorant(s) direct(s) de formule A-L-B par rapport au poids total de la composition.

La composition tinctoriale conforme à l'invention peut en outre contenir un ou plusieurs colorants directs additionnels autres que les colorants directs de formule A-L-B décrits ci-dessus, pouvant notamment être choisis parmi les colorants directs mentionnés plus haut, et notamment parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

On peut ainsi utiliser les colorants choisis parmi les colorants nitro aromatiques, anthraquinoniques, naphtoquinoniques, benzoquinoniques, naphtoquinoniques, azoïques, xanthéniques, triarylméthaniques, aziniques, thiaziniques, phénothiaziniques, diaziniques, phénodiaziniques, acridiniques, cyanineméthiniques, azométhiniques, nitrés, phtalocyaniques, indoaniliques, indophénoliques, et indoaminiques, et les colorants directs naturels.

Parmi les colorants directs benzéniques utilisables selon l'invention, on peut citer de manière non limitative les composés suivants :
- 1,4-diamino-2-nitrobenzène,
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1- β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention, on peut citer les colorants azoïques cationiques décrits dans les demandes de brevets WO 95/15144, WO-95/01772 et EP-714954 dont le contenu fait partie intégrante de l'invention.

Parmi ces composés, on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs additionnels représentent de préférence de 0,001 à 20%, et encore plus préférentiellement de 0,01 à 10% en poids du poids total de la composition prête à l'emploi.

A titre d'exemple, les bases d'oxydation pouvant être présentes dans les composition selon l'invention sont choisies parmi les phénylènediamines, les bis-phénylalkylènediamines, les para-aminophénols, les ortho-aminophénols, les bases hétérocycliques autres que les paraphénylènediamines hétérocycliques de formule (I) et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4,5-diamino-1-(β-triéthoxyéthyl)pyrazole.

La ou les bases d'oxydation présentes dans la composition de l'invention sont en général présentes en quantité comprise allant de 0,001 à 20 % en poids environ du poids total de la composition tinctoriale, de préférence allant de 0,005 à 6 %.

La composition de la présente invention peut en outre comprendre un ou plusieurs précurseurs de colorant d'oxydation choisis parmi les coupleurs.

Les coupleurs peuvent être choisis parmi les coupleurs conventionnellement utilisés pour la teinture de fibres kératiniques. Parmi ces coupleurs, on peut notamment citer les métaphénylènediamines, les métadiphénols, les coupleurs naphtaléniques, les coupleurs hétérocycliques ainsi que leur sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en une quantité allant de 0,001 à 20 % en poids du poids total de la composition tinctoriale, de préférence allant de 0,01 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensioactifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium :

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La mise en oeuvre du procédé selon la présente demande comprend l'application d'une composition tinctoriale selon la présente demande sur les fibres kératiniques, puis une étape consistant à laisser poser pendant une période suffisante pour permettre la coloration des cheveux, cette période est généralement comprise entre 5 minutes et 1 heure et de préférence entre 15 minutes et 1 heure.

Le procédé de teinture des fibres kératiniques peut comprendre une étape mettant en oeuvre un agent oxydant à pH acide, neutre ou alcalin. L'agent oxydant peut être ajouté à la composition de l'invention juste au moment de l'emploi ou il peut être mis en oeuvre à partir d'une composition oxydante le contenant, appliqué simultanément ou séquentiellement à la composition de l'invention.

Dans une variante de ce procédé, la composition comprenant le ou les composés de formule A-L-B contient au moins un précurseur de colorant d'oxydation.

Selon un mode de réalisation particulier, la composition selon la présente invention comprenant le composé de formule A-L-B et éventuellement au moins un précurseur de colorant d'oxydation est mélangée, de préférence au moment de l'emploi, à une composition contenant, dans un milieu approprié pour la teinture, au moins un agent oxydant. Le mélange obtenu est ensuite appliqué sur les fibres kératiniques. Après un temps de pause de 5 minutes à 1 heure environ, de préférence 15 minutes à 1 heure environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et de préférence entre 5 et 11 environ et de manière encore plus préférée entre 6 et 8,5. Il peut être ajusté à la valeur désirée au moyen d'agents régulateurs de pH, acidifiants ou alcalinisants habituellement utilisés en teinture des fibres kératiniques et tels que définis précédemment.

La composition appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

La présente demande a également pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour obtenir la coloration désirée.

Les exemples qui suivent sont destinés à illustrer l'invention, sans toutefois présenter un caractère limitatif.

### Exemples de préparations de colorants utilisés selon l'invention

Les produits revendiqués sont élaborés par une suite de réactions connues de l'homme de l'art.

### Exemple 1 : Synthèse du dichlorure de 4-{4-[(E)-(1,3-diméthyl-1H-imidazol-3-ium-2-yl)diazényl]phényl}-1-[6-(4-{4-[(E)-(1,3-diméthyl-1H-imidazol-3-ium-2-yl)diazényl]phényl}-1-méthylpipérazine-1-ium-1-yl)hexyl]-1-méthylpipérazine-1-ium bis(méthylsulfate) (II)

### Etape 1

Synthèse du chlorure de 2-[(E)-(4-{4-[6-(4-{4-[(E)-(1,3-diméthyl-1H-imidazol-3-ium-2-yl)diazényl]phényl}pipérazine-1-yl)hexyl]pipérazine-1-yl}phényl)diazényl]-1,3-diméthyl-1H-imidazol-3-ium (I)

Le chlorure de 1.3-diméthylique-2-[(E)-(4-pipérazine-1-ylphényl)diazényl]-1H-imidazole-3-ium (0.9g) a été solubilisé en DMF (20ml), Br(CH₂)₆Br (0.5 mol eq) et carbonate de potassium (1 mol eq). Le mélange réactionnel a été agité à 80°C pendant 24h puis refroidi à température ambiante et filtré. Le produit brut a été précipité dans l'éther diisopropylique (200ml) et purifié par la chromatographie.

On suit la réaction par chromatographie sur couches minces (CCM) : phase normale Silice *:éluant CH2Cl2 :éthanol :tampon pH9 (1:8:1)*.

### Etape 2

Le chlorure de 2-[(E)-(4-{4-[6-(4-{4-[(E)-(1,3-dimethyl-1H-imidazol-3-ium-2-yl)diazenyl]phényl}pipérazine-1-yl)hexyl]pipérazine-1-yl}phényle)diazényle]-1,3-diméthyle-1H-imidazol-3-ium (I, 0.3g) a été solubilisé par le DMF (20ml) et le diméthylsulfate (2.2mol eq) et agité à 80°C pendant 6h. Le produit brut a été précipité dans l'éther diisopropylique (200ml) et purifié par la chromatographie.

Suivi de réaction par CCM : phase normale Silice *:éluant CH2Cl2 :éthanol :tampon pH9 (1:8:1)*.

### Exemple 2 : Synthèse du tétrachlorure de 2-{(E)-[4-({3-[3-(6-{1-[3-({4-[(E)-(1,3-diméthyl-1H-imidazol-3-ium-2-yl)diazenyl]phényl}amino)propyl]-1H-imidazol-3-ium-3-yl}hexyl)-1H-imidazol-3-ium-1-yl]propyl}amino)phényle]diazényl}-1,3-diméthyl-1H-imidazol-3-ium (III)

### Etape 1

Le chlorure de 2-[(E)-(4-{[3-(1H-imidazol-1-yl)propyl]amino}phényl)diazényl]-1,3-diméthyle-1H-imidazol-3-ium (1g) était solubilisé par le DMF (20ml) et Br(CH₂)₆Br (0.5 mol eq). Le mélange réactionnel a été agité à 80°C pendant 24h puis refroidi à température ambiante. Le produit brut a été précipité dans l'éther diisopropylique (200ml) et purifié par la chromatographie. L'étape finale réside en un échange d'ions classique sur résine activé par l'acide hydrochlorique.

Suivi de réaction : CCM : phase normale Silice *:éluant CH2Cl2 :éthanol :tampon pH9 (1:8:1)*.

| **Exemples de compositions tinctoriales** | | |
|---|---|---|
| | A | B |
| Colorant de formule (II) | 0,1 | - |
| Colorant de formule (III) | - | 0,1 |
| Alkyl(C8-C10)polyglucoside (pourcentage de matière active) | 3 | 3 |
| Ethanol | 10 | 10 |
| 2-amino-2-méthyl-1-propanol | Qsp pH 8,5 | Qsp pH 8,5 |
| Eau | Qsp 100g | Qsp 100g |

Chacune des compositions A et B est appliquée sur les cheveux pendant 30 minutes. Après rinçage et séchage, les cheveux sont colorés dans une nuance rouge intense et peu sélective.

### Exemple 3 : Synthèse du sel de dichlorure et de méthosulfate de 2-{(E)-[4-({3-[[3-({4-[(E)-(1,3-diméthyl-1H-imidazol-3-ium-2-yl)diazényl]phényl}amino)propyl](diméthyl)ammonio]propyl}amino)p hényl]diazényl}-1,3-diméthyl-1H-imidazol-3-ium (IV)

### Etape 1 :

Le chlorure de 2-[(E)-(4-méthoxyphényl)diazényl]-1,3-diméthyl-1H-imidazol-3-ium (10 g, 0.0375 mole) est solubilisé dans l'isopropanol (30ml) et l'eau (20 ml). 2,72 g (0.0187 mole) de N,N-bis(3-aminopropyl)-N-méthylamine sont alors ajoutés au milieu réactionnel. Celui-ci est agité à 80°C, pendant 12 heures puis refroidi à température ambiante. Le produit brut précipite dans l'isopropanol. Cependant, ce brut est recristallisé plusieurs fois dans l'éthanol. Le précipité obtenu est filtré puis séché sous vide.

Suivi de réaction : CCM : phase normale Silice : *éluant CH2Cl2 : éthanol : tampon pH9 (1:8:1)*.

Les analyses sont conformes au produit attendu.

### Etape 2 :

Le dichlorure de 2-{(E)-[4-({3-[[3-({4-[(E)-(1,3-dimethyl-1H-imidazol-3-ium-2-yl)diazényl]phényl}amino)propyl](méthyl)amino]propyl}amino)phényl ]diazényl}-1,3-diméthyl-1H-imidazol-3-ium (1,5 g) est solubilisé dans la diméthylformamide (23 ml). 0,38 ml de diméthylsulfate est alors ajouté au milieu réactionnel. Celui-ci est agité à 50°C, pendant 22 heures puis refroidi à température ambiante. Le produit brut attendu est précipité dans une solution d'acétate d'éthyle, filtré puis séché sous vide.

Suivi de réaction : CCM : phase normale Silice : *éluant n-BuOH : H₂O : AcOH (40:30:15).*

Les analyses sont conformes au produit attendu.

L'application de ce colorant conduit à une coloration des cheveux dans une nuance rouge intense et peu sélective.

## Revendications

1. Utilisation à titre de colorant direct dans des compositions tinctoriales pour fibres kératiniques, en particulier les fibres kératiniques humaines telles que les cheveux, ou pour la fabrication de telles compositions, d'un composé de formule A-L-B, dans laquelle A et B désignent un groupe à fonction colorante de type arylazoimidazolium, et L désigne un bras de liaison comprenant au moins un groupe cationique C.

2. Utilisation selon la revendication 1, **caractérisée en ce que** le groupe C est choisi parmi les groupes cationiques aliphatiques ou hétérocycliques.

3. Utilisation selon la revendication 2, **caractérisée en ce que** le groupe cationique hétérocyclique est choisi parmi les groupements imidazolium, pipéridinium, pyridinium, pyrazolium, et triazolium.

4. Utilisation selon la revendication 3, **caractérisée en ce que** le groupe cationique hétérocyclique est un groupement imidazolium.

5. Utilisation selon la revendication 2, **caractérisée en ce que** le groupe cationique aliphatique est choisi parmi les radicaux divalents du type -N⁺-R₁R₂-, R₁ et R₂ désignant indépendamment l'un de l'autre un radical alkyle en C₁-C₁₀, mono ou polyhydroxyalkyle en C₁-C₁₀.

6. Utilisation selon l'une des revendicatoins 1 à 5, **caractérisée en ce que** le bras de liaison L est une chaîne hydrocarbonée, linéaire ou ramifiée, saturée ou insaturée, pouvant être terminée ou interrompue par un ou plusieurs groupements carbonyle et/ou par un ou plusieurs hétéroatomes, ou une chaîne comprenant un ou plusieurs cycles aromatiques ou un ou plusieurs hétérocycles aromatiques ou saturés.

7. Utilisation selon la revendication 6, **caractérisée en ce que** la chaîne hydrocarbonée est substituée par un radical hydroxy, alcoxy, amino, alkylamino ou un halogène.

8. Utilisation selon la revendication 6 ou 7, **caractérisée en ce que** le bras de liaison L est une chaîne hydrocarbonée en C₁-C₄₀, plus particulièrement en C₁-C₂₀, de préférence en C₁-C₈, linéaire ou ramifiée.

9. Utilisation selon l'une des revendications 1 à 8, **caractérisée en ce que** le composé de formule A-L-B est choisi parmi les composés de formule (I) suivante : 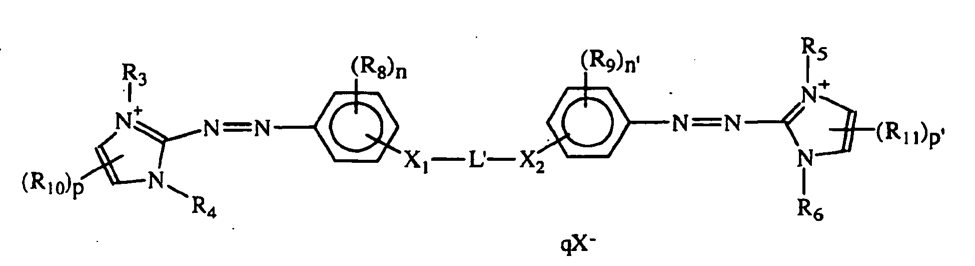 dans laquelle :
X₁, X₂ désignent indépendamment l'un de l'autre un cycle pipérazine substitué par un radical alkyle en C₁-C₈, un radical -O- ou -NR₇-, R₇ désignant un atome d'hydrogène, un radical alkyle en C₁-C₈, ou un radical hydroxyalkyle en C₁-C₈;
R₃ à R₆ désignent indépendamment les uns des autres un radical alkyle en C₁-C₈, ou un radical hydroxyalkyle en C₁-C₈;
R₈ et R₉ désignent un atome d'hydrogène, un radical alkyle en C₁-C₄ éventuellement substitué par un ou plusieurs radicaux choisis parmi les radicaux hydroxy, amino, (di)alkylamino en C₁-C₂; un radical carboxy ; un radical alcoxy en C₁-C₂; un radical amino ; un radical (di)alkylamino en C₁-C₂ ; un radical (poly)-hydroxyalkylamino en C₂-C₄ ;
R₁₀ et R₁₁ désignent, indépendamment l'un de l'autre, un atome d'hydrogène, un radical alkyle en C₁-C₄ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ; un radical phényle éventuellement substitué ; un radical carboxy ; un radical sulfonylamino ;
L' est un bras de liaison ; le groupement X₁-L'-X₂ comprenant au moins un groupe cationique C ;
n et n' désignent un nombre entier allant de 0 à 4 ;
p et p' désignent un nombre entier allant de 0 à 2 ;
q désigne un nombre entier allant de 3 à 50, de préférence de 3 à 10, et encore plus préférentiellement de 3 à 5 ;
X⁻ désigne un anion d'origine minérale ou organique.

10. Utilisation selon la revendication 9, **caractérisée en ce que** q est choisi de façon à assurer la neutralité des charges du composé de formule (I).

11. Utilisation selon la revendication 9, **caractérisée en ce que** X⁻ est choisi parmi les ions halogénures, les ions sulfate ou hydrogénosulfate, les ions méthosulfate, tosylate, carbonate ou hydrogénocarbonate, phosphate, nitrate, citrate.

12. Utilisation selon l'une des revendications 1 à 11, **caractérisée en ce que** le composé de formule A-L-B est choisi parmi les composés de formules suivantes : 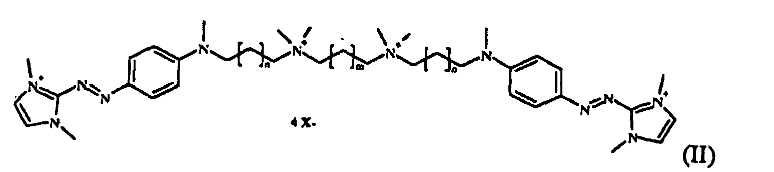 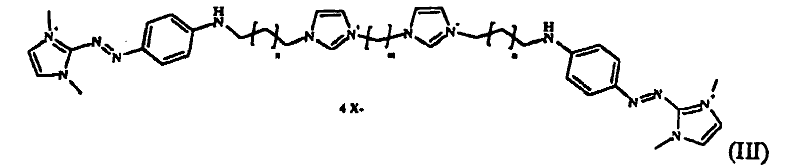 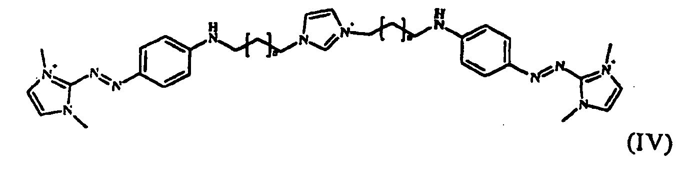 dans lesquelles :
X⁻ désigne un anion d'origine minérale ou organique, et
m et n sont des entiers allant de 0 à 20 et de préférence de 0 à 8 ;
m et n étant tels que le bras de liaison comprend de 1 à 40, plus particulièrement de 1 à 20, de préférence de 1 à 8 atomes de carbone.

13. Composition tinctoriale pour la teinture des fibres kératiniques, en particulier des fibres kératiniques humaines telles que les cheveux comprenant dans un milieu approprié pour la teinture au moins un composé de formule A-L-B, dans laquelle A et B désignent un groupe à fonction colorante de type arylazoimidazolium, et L désigne un bras de liaison comprenant au moins un groupe cationique C, et au moins une base d'oxydation et/ou au moins un colorant direct additionnel.

14. Composition selon la revendication 13, **caractérisée en ce que** le groupe C est choisi parmi les groupes cationiques aliphatiques ou hétérocycliques.

15. Composition selon la revendication 13, **caractérisée en ce que** le groupe cationique hétérocyclique est choisi parmi les groupements imidazolium, pipéridinium, pyridinium, pyrazolium, et triazolium.

16. Composition selon la revendication 14, **caractérisée en ce que** le groupe cationique hétérocyclique est un groupement imidazolium.

17. Composition selon la revendication 14, **caractérisée en ce que** le groupe cationique aliphatique est choisi parmi les radicaux divalents du type -N⁺-R₁R₂-, R₁ et R₂ désignant indépendamment l'un de l'autre un radical alkyle en C₁-C₁₀, mono ou polyhydroxyalkyle en C₁-C₁₀.

18. Composition selon la revendication 13, **caractérisée en ce que** le bras de liaison est une chaîne hydrocarbonée, linéaire ou ramifiée, saturée ou insaturée, pouvant être terminée ou interrompue par un ou plusieurs groupements carbonyle et/ou par un ou plusieurs hétéroatomes, ou une chaîne comprenant un ou plusieurs cycles aromatiques ou un ou plusieurs hétérocycles aromatiques ou saturés.

19. Composition selon la revendication 18, **caractérisée en ce que** la chaîne hydrocarbonée est substituée par un radical hydroxy, alcoxy, amino, alkylamino ou un halogène.

20. Composition selon la revendication 18 ou 19, **caractérisée en ce que** le bras de liaison L est une chaîne hydrocarbonée C₁-C₄₀, plus particulièrement en C₁-C₂₀, de préférence en C₁-C₈, linéaire ou ramifiée.

21. Composition selon l'une des revendications 13 à 20, **caractérisée en ce que** le composé de formule A-L-B est choisi parmi les composés de formule (I) suivante : 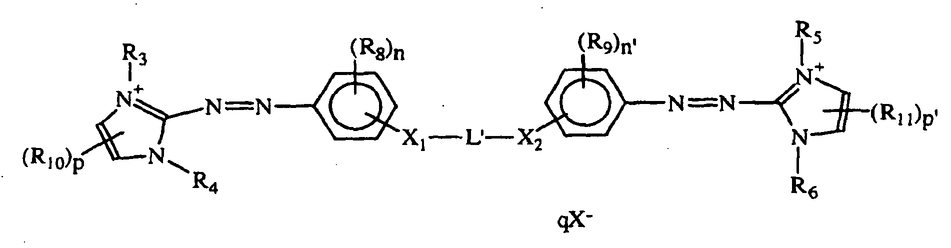 dans laquelle :
X₁, X₂ désignent indépendamment l'un de l'autre un cycle pipérazine substitué par un radical alkyle en C₁-C₈, un radical -O- ou -NR₇, R₇ désignant un atome d'hydrogène, un radical alkyle en C₁-C₈, ou un radical hydroxyalkyle en C₁-C₈;
R₃ à R₆ désignent indépendamment les uns des autres un radical alkyle en C₁-C₈, ou un radical hydroxyalkyle en C₁-C₈;
R₈ et R₉ désignent un atome d'hydrogène, un radical alkyle en C₁-C₄ éventuellement substitué par un ou plusieurs radicaux choisis parmi les radicaux hydroxy, amino, (di)alkylamino en C₁-C₂ ; un radical carboxy ; un radical alcoxy en C₁-C₂ ; un radical amino ; un radical (di)alkylamino en C₁-C₂ ; un radical (poly)-hydroxyalkylamino en C₂-C₄ ;
R₁₀ et R₁₁ désignent, indépendamment l'un de l'autre, un atome d'hydrogène, un radical alkyle en C₁-C₄ linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ; un radical phényle éventuellement substitué ; un radical carboxy ; un radical sulfonylamino ;
L' est un bras de liaison ; le groupement X₁-L'-X₂ comprenant au moins un groupe cationique C ;
n et n' désignent un nombre entier allant de 0 à 4 ;
p et p' désignent un nombre entier allant de 0 à 2 ;
q désigne un nombre entier allant de 3 à 50, de préférence de 3 à 10, et encore plus préférentiellement de 3 à 5 ;
X⁻ désigne un anion d'origine minérale ou organique.

22. Composition selon la revendication 21, **caractérisée en ce que** q est choisi de façon à assurer la neutralité des charges du composé de formule (I).

23. Composition selon la revendication 21, **caractérisée en ce que** X⁻ est choisi parmi les ions halogénures, les ions sulfate ou hydrogénosulfate, les ions méthosulfate, tosylate, carbonate ou hydrogénocarbonate, phosphate, nitrate, citrate.

24. Composition selon l'une des revendications 13 à 23, **caractérisée en ce que** le composé de formule A-L-B est choisi parmi les composés de formules suivantes : 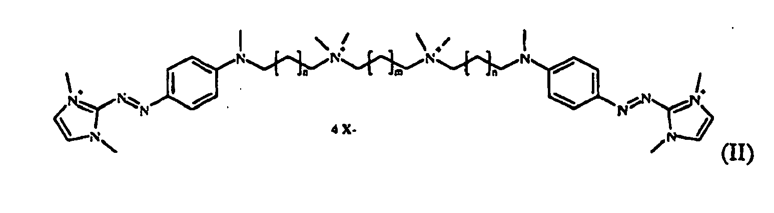 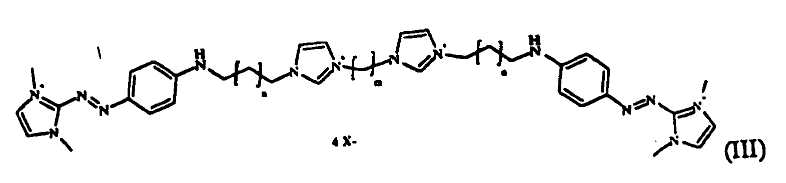 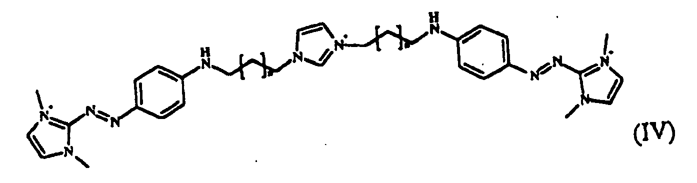 dans lesquelles :
X⁻ désigne un anion d'origine minérale ou organique, et
m et n sont des entiers allant de 0 à 20 et de préférence de 0 à 8 ;
m et n étant tels que le bras de liaison comprend de 1 à 40, plus particulièrement de 1 à 20, de préférence de 1 à 8 atomes de carbone.

25. Composition selon l'une des revendications 13 à 24, **caractérisée en ce qu'**elle comprend de 0,001 à 20%, de préférence de 0,01 à 10%, et de manière encore préférée de 0,1 à 5% en poids de colorant(s) direct(s) de formule A-L-B par rapport au poids total de la composition.

26. Composition tinctoriale selon l'une des revendications 13 à 25, **caractérisée en ce que** la base d'oxydation est choisie parmi les paraphénylènediamines, les bis-phénylalkylènediamines, les paraaminophénols, les orthoaminophénols, les bases hétérocycliques et leurs sels d'addition.

27. Composition tinctoriale selon l'une des revendications 13 à 26, **caractérisée en ce que** la ou les bases d'oxydation sont présentes en une quantité comprise entre 0,001 à 20 % en poids et de préférence entre 0,005 et 6 % en poids par rapport au poids total de la composition.

28. Composition selon l'une des revendications 13 à 27, **caractérisée en ce que** le colorant direct additionnel est choisi parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

29. Composition selon la revendication 28, **caractérisée en ce que** le colorant direct additionnel représente de 0,001 à 20%, et de préférence de 0,01 à 10% en poids du poids total de la composition.

30. Composition selon l'une des revendications 13 à 29, **caractérisée en ce qu'**elle contient au moins un précurseur de colorant d'oxydation choisi parmi les coupleurs.

31. Composition selon la revendication 30, **caractérisée en ce que** le coupleur est choisi parmi les métaphénylènediamines, les métaaminophénols, les métadiphénols, les coupleurs naphtaléniques, les coupleurs hétérocycliques et leurs sels d'addition.

32. Composition selon la revendication 30, **caractérisée en ce que** le coupleur est choisi parmi le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

33. Composition selon l'une des revendications 30 à 32, **caractérisée en ce que** le ou les coupleurs sont présents en une quantité comprise entre 0,001 et 20 % de préférence entre 0,005 et 6 % en poids par rapport au poids total de la composition.

34. Composition selon l'une des revendications 13 à 33, **caractérisée en ce qu'**elle comprend au moins un adjuvant choisi parmi les tensioactifs anioniques, cationiques, non ioniques, amphotères, zwitterioniques ou leurs mélanges, les polymères anioniques, cationiques, non ioniques, amphotères, zwitterioniques ou leurs mélanges, les agents épaississants minéraux ou organiques, les agents antioxydants, les agents de pénétration, les agents séquestrants, les parfums, les tampons, les agents dispersants, les agents conditionneurs, les agents filmogènes, les céramides, les agents conservateurs, les agents opacifiants.

35. Composition selon l'une des revendications 13 à 34, **caractérisée en ce qu'**elle comprend au moins un agent oxydant choisi parmi le peroxyde d'hydrogène, le peroxyde d'urée, les bromates de métaux alcalins, les persels, les peracides et les enzymes oxydases.

36. Composition selon la revendication 35, **caractérisée en ce que** l'agent oxydant est le peroxyde d'hydrogène.

37. Procédé de teinture des fibres kératiniques, **caractérisé en ce qu'**il comprend l'application d'une composition selon l'une des revendications 13 à 36, sur les fibres kératiniques, puis une étape consistant à laisser poser la composition.

38. Utilisation d'une composition selon l'une des revendications 13 à 36 pour la teintures des fibres kératiniques, en particulier des fibres kératiniques humaines telles que les cheveux.

39. Utilisation d'une composition selon l'une des revendications 13 à 36 sur des fibres kératiniques, en particulier les fibres kératiniques humaines telles que les cheveux, pour obtenir des teintures présentant une bonne résistance aux agents extérieurs et aux shampoings.

## Claims

1. Use, as a direct dye in dye compositions for keratin fibres, in particular human keratin fibres such as the hair, or for the manufacture of such compositions, of a compound of formula A-L-B, in which A and B denote a colouring function of arylazoimidazolium type and L denotes a linker comprising at least one cationic group C.

2. Use according to Claim 1, **characterized in that** the group C is chosen from aliphatic and heterocyclic cationic groups.

3. Use according to Claim 2, **characterized in that** the heterocyclic cationic group is chosen from imidazolium, piperidinium, pyridinium, pyrazolium and triazolium groups.

4. Use according to Claim 3, **characterized in that** the heterocyclic cationic group is an imidazolium group.

5. Use according to Claim 2, **characterized in that** the aliphatic cationic group is chosen from divalent radicals of the type -N⁺-R₁R₂-, R₁ and R₂ denoting, independently of each other, a C₁-C₁₀ alkyl or a C₁-C₁₀ mono- or polyhydroxyalkyl radical.

6. Use according to one of Claims 1 to 5, **characterized in that** the linker L is a linear or branched, saturated or unsaturated hydrocarbon-based chain, possibly ending or interrupted with one or more carbonyl groups and/or with one or more hetero atoms, or a chain comprising one or more aromatic rings or one or more aromatic or saturated heterocycles.

7. Use according to Claim 6, **characterized in that** the hydrocarbon-based chain is substituted with a hydroxyl, alkoxy, amino or alkylamino radical or a halogen.

8. Use according to Claim 6 or 7, **characterized in that** the linker L is a linear or branched C₁-C₄₀, more particularly C₁-C₂₀ and preferably C₁-C₈ hydrocarbon-based chain.

9. Use according to one of Claims 1 to 8, **characterized in that** the compound of formula A-L-B is chosen from the compounds of formula (I) below: 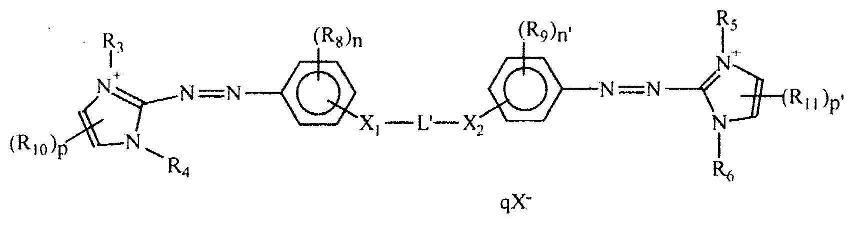 in which:
X₁ and X₂ denote, independently of each other, a piperazine ring substituted with a C₁-C₈ alkyl radical, a radical -O- or -NR₇-, R₇ denoting a hydrogen atom, a C₁-C₈ alkyl radical or a C₁-C₈ hydroxyalkyl radical;
R₃ to R₆ denote, independently of each other, a C₁-C₈ alkyl radical or a C₁-C₈ hydroxyalkyl radical;
R₈ and R₉ denote a hydrogen atom, a C₁-C₄ alkyl radical optionally substituted with one or more radicals chosen from hydroxyl, amino and C₁-C₂ (di)alkylamino radicals; a carboxyl radical; a C₁-C₂ alkoxy radical; an amino radical; a C₁-C₂ (di)alkylamino radical; a C₂-C₄ (poly)hydroxy-alkylamino radical;
R₁₀ and R₁₁ denote, independently of each other, a hydrogen atom, a linear or branched C₁-C₄ alkyl radical optionally substituted with one or more radicals chosen from hydroxyl, C₁-C₂ alkoxy, C₂-C₄ (poly)hydroxyalkoxy, amino, C₁-C₂ (di)alkylamino, carboxyl and sulfonic radicals; an optionally substituted phenyl radical; a carboxyl radical; a sulfonylamino radical;
L' is a linker; the group X₁-L'-X₂ comprising at least one cationic group C;
n and n' denote an integer ranging from 0 to 4;
p and p' denote an integer ranging from 0 to 2;
q denotes an integer ranging from 3 to 50, preferably from 3 to 10 and even more preferably from 3 to 5;
X⁻ denotes an anion of mineral or organic origin.

10. Use according to Claim 9, **characterized in that** q is chosen so as to ensure the neutrality of the charges of the compound of formula (I).

11. Use according to Claim 9, **characterized in that** X⁻ is chosen from halide ions, sulfate or hydrogen sulfate ions, methosulfate ions, tosylate ions, carbonate or hydrogen carbonate ions, phosphate ions, nitrate ions and citrate ions.

12. Use according to one of Claims 1 to 11, **characterized in that** the compound of formula A-L-B is chosen from the compounds having the following formulae: 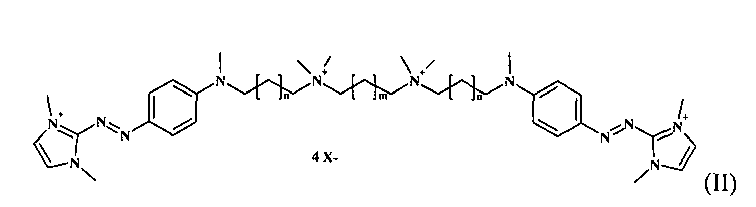 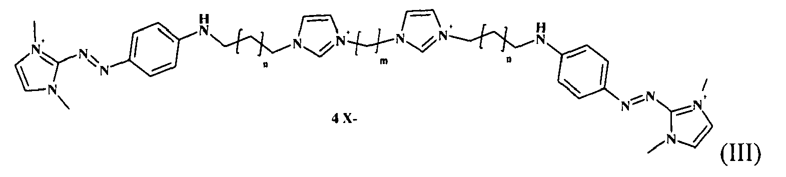 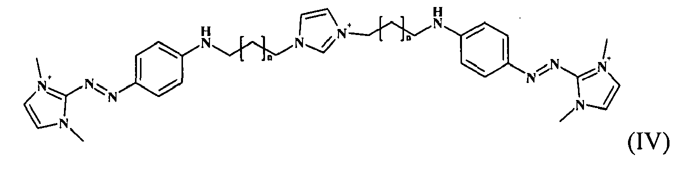 in which:
X⁻ denotes an anion of mineral or organic origin, and
m and n are integers ranging from 0 to 20 and preferably from 0 to 8;
m and n being such that the linker contains from 1 to 40, more particularly from 1 to 20 and preferably from 1 to 8 carbon atoms.

13. Dye composition for dyeing keratin fibres, in particular human keratin fibres such as the hair, comprising, in a medium that is suitable for dyeing, at least one compound of formula A-L-B, in which A and B denote a group containing a colouring function of arylazoimidazolium type, and L denotes a linker comprising at least one cationic group C, and at least one oxidation base and/or at least one additional direct dye.

14. Composition according to Claim 13, **characterized in that** the group C is chosen from aliphatic and heterocyclic cationic groups.

15. Composition according to Claim 13, **characterized in that** the heterocyclic cationic group is chosen from imidazolium, piperidinium, pyridinium, pyrazolium and triazolium groups.

16. Composition according to Claim 14, **characterized in that** the heterocyclic cationic group is an imidazolium group.

17. Composition according to Claim 14, **characterized in that** the aliphatic cationic group is chosen from divalent radicals of the type -N⁺-R₁R₂-, R₁ and R₂ denoting, independently of each other, a C₁-C₁₀ alkyl or a C₁-C₁₀ mono- or polyhydroxyalkyl radical.

18. Composition according to Claim 13, **characterized in that** the linker L is a linear or branched, saturated or unsaturated hydrocarbon-based chain, possibly ending or interrupted with one or more carbonyl groups and/or with one or more hetero atoms, or a chain comprising one or more aromatic rings or one or more aromatic or saturated heterocycles.

19. Composition according to Claim 18, **characterized in that** the hydrocarbon-based chain is substituted with a hydroxyl, alkoxy, amino or alkylamino radical or a halogen.

20. Composition according to Claim 18 or 19, **characterized in that** the linker L is a linear or branched C₁-C₄₀, more particularly C₁-C₂₀ and preferably C₁-C₈ hydrocarbon-based chain.

21. Composition according to one of Claims 13 to 20, **characterized in that** the compound of formula A-L-B is chosen from the compounds of formula (I) below: 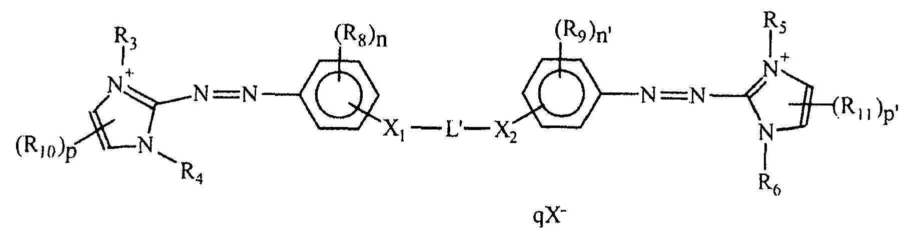 in which:
X₁ and X₂ denote, independently of each other, a piperazine ring substituted with a C₁-C₈ alkyl radical; a radical -O- or -NR₇-, R₇ denoting a hydrogen atom, a C₁-C₈ alkyl radical or a C₁-C₈ hydroxyalkyl radical;
R₃ to R₆ denote, independently of each other, a C₁-C₈ alkyl radical or a C₁-C₈ hydroxyalkyl radical;
R₈ and R₉ denote a hydrogen atom, a C₁-C₄ alkyl radical optionally substituted with one or more radicals chosen from hydroxyl, amino and C₁-C₂ (di)alkylamino radicals; a carboxyl radical; a C₁-C₂ alkoxy radical; an amino radical; a C₁-C₂ (di)alkylamino radical; a C₂-C₄ (poly)hydroxyalkylamino radical;
R₁₀ and R₁₁ denote, independently of each other, a hydrogen atom, a linear or branched C₁-C₄ alkyl radical optionally substituted with one or more radicals chosen from hydroxyl, C₁-C₂ alkoxy, C₂-C₄ (poly)hydroxyalkoxy, amino, C₁-C₂ (di)alkylamino, carboxyl and sulfonic radicals; an optionally substituted phenyl radical; a carboxyl radical; a sulfonylamino radical;
L' is a linker; the group X₁-L'-X₂ comprising at least one cationic group C;
n and n' denote an integer ranging from 0 to 4;
p and p' denote an integer ranging from 0 to 2;
q denotes an integer ranging from 3 to 50, preferably from 3 to 10 and even more preferably from 3 to 5;
X- denotes an anion of mineral or organic origin.

22. Composition according to Claim 21, **characterized in that** q is chosen so as to ensure the neutrality of the charges of the compound of formula (I).

23. Composition according to Claim 21, **characterized in that** X⁻ is chosen from halide ions, sulfate or hydrogen sulfate ions, methosulfate ions, tosylate ions, carbonate or hydrogen carbonate ions, phosphate ions, nitrate ions and citrate ions.

24. Composition according to one of Claims 13 to 23, **characterized in that** the compound of formula A-L-B is chosen from the compounds having the following formulae: 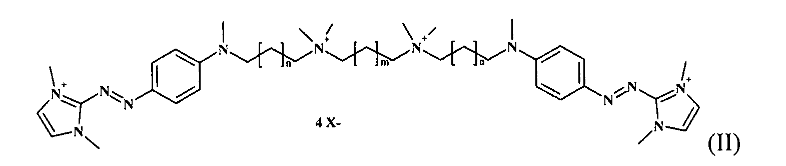 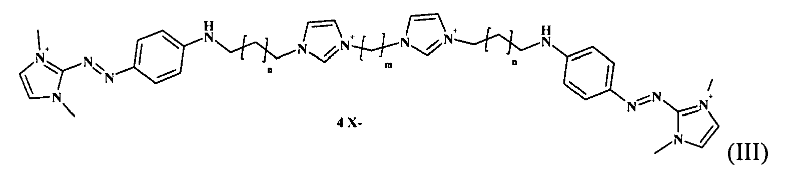 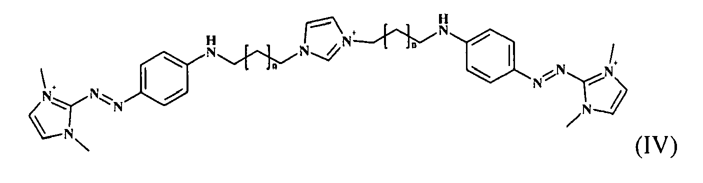 in which:
X⁻ denotes an anion of mineral or organic origin, and
m and n are integers ranging from 0 to 20 and preferably from 0 to 8;
m and n being such that the linker contains from 1 to 40, more particularly from 1 to 20 and preferably from 1 to 8 carbon atoms.

25. Composition according to one of Claims 13 to 24, **characterized in that** it comprises from 0.001% to 20%, preferably from 0.01% to 10% and even more preferably from 0.1% to 5% by weight of direct dye(s) of formula A-L-B relative to the total weight of the composition.

26. Dye composition according to one of Claims 13 to 25, **characterized in that** the oxidation base is chosen from para-phenylenediamines, bis(phenyl)alkylenediamines, para-aminophenols, ortho-aminophenols and heterocyclic bases, and the addition salts thereof.

27. Dye composition according to one of Claims 13 to 26, **characterized in that** the oxidation base(s) is (are) present in an amount of between 0.001% and 20% by weight and preferably between 0.005% and 6% by weight relative to the total weight of the composition.

28. Composition according to one of Claims 13 to 27, **characterized in that** the additional direct dye is chosen from neutral, acidic or cationic nitrobenzene dyes, neutral, acidic or cationic azo direct dyes, neutral, acidic or cationic quinone and in particular anthraquinone direct dyes, azine direct dyes, triarylmethane direct dyes, indoamine direct dyes and natural direct dyes.

29. Composition according to Claim 28, **characterized in that** the additional direct dye represents from 0.001% to 20% and preferably from 0.01% to 10% by weight relative to the total weight of the composition.

30. Composition according to one of Claims 13 to 29, **characterized in that** it contains at least one oxidation dye precursor chosen from couplers.

31. Composition according to Claim 30, **characterized in that** the coupler is chosen from meta-phenylenediamines, meta-aminophenols, meta-diphenols, naphthalene-based couplers and heterocyclic couplers, and the addition salts thereof.

32. Composition according to Claim 30, **characterized in that** the coupler is chosen from 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene and 2,6-bis(β-hydroxyethylamino)toluene, and the addition salts thereof.

33. Composition according to one of Claims 30 to 32, **characterized in that** the coupler(s) is (are) present in an amount of between 0.001% and 20% and preferably between 0.005% and 6% by weight relative to the total weight of the composition.

34. Composition according to one of Claims 13 to 33, **characterized in that** it comprises at least one adjuvant chosen from anionic, cationic, nonionic, amphoteric or zwitterionic surfactants or mixtures thereof, anionic, cationic, nonionic, amphoteric or zwitterionic polymers or mixtures thereof, mineral or organic thickeners, antioxidants, penetrating agents, sequestering agents, fragrances, buffers, dispersants, conditioners, film-forming agents, ceramides, preserving agents and opacifiers.

35. Composition according to one of Claims 13 to 34, **characterized in that** it comprises at least one oxidizing agent chosen from hydrogen peroxide, urea peroxide, alkali metal bromates, persalts, peracids and oxidase enzymes.

36. Composition according to Claim 35, **characterized in that** the oxidizing agent is hydrogen peroxide.

37. Process for dyeing keratin fibres, **characterized in that** it comprises the application of the composition according to one of Claims 13 to 36 to the keratin fibres, followed by a step consisting in leaving the composition on the fibres.

38. Use of a composition according to one of Claims 13 to 36 for dyeing keratin fibres, in particular human keratin fibres such as the hair.

39. Use of a composition according to one of Claims 13 to 36 on keratin fibres, in particular human keratin fibres such as the hair, to obtain dyeing results that show good resistance to external agents and to shampoos.

## Patentansprüche

1. Verwendung einer Verbindung der Formel A-L-B, worin A und B für eine Gruppe mit Färbefunktion vom Arylazoimidazolium-Typ stehen und L für eine Brückengruppe mit mindestens einer kationischen Gruppe C steht, als Direktfarbstoff in Färbezusammensetzungen für Keratinfasern, insbesondere menschliche Keratinfasern wie die Haare, oder zur Herstellung derartiger Zusammensetzungen.

2. Verwendung nach Anspruch 1, **dadurch gekennzeichnet, daß** die Gruppe C unter aliphatischen oder heterocyclischen kationischen Gruppen ausgewählt ist.

3. Verwendung nach Anspruch 2, **dadurch gekennzeichnet, daß** die heterocyclische kationische Gruppe unter Imidazolium-, Piperidinium-, Pyridinium-, Pyrazolium- und Triazoliumgruppen ausgewählt ist.

4. Verwendung nach Anspruch 3, **dadurch gekennzeichnet, daß** es sich bei der heterocyclischen kationischen Gruppe um eine Imidazoliumgruppe handelt.

5. Verwendung nach Anspruch 2, **dadurch gekennzeichnet, daß** die aliphatische kationische Gruppe unter zweiwertigen Resten des Typs -N⁺-R₁R₂-, wobei R₁ und R₂ unabhängig voneinander für einen Alkylrest mit 1 bis 10 Kohlenstoffatomen oder einen Monohydroxyalkyl- oder Polyhydroxyalkylrest mit 1 bis 10 Kohlenstoffatomen stehen, ausgewählt ist.

6. Verwendung nach einem der Ansprüche 1 bis 5, **dadurch gekennzeichnet, daß** es sich bei der Brückengruppe L um eine gesättigte oder ungesättigte, lineare oder verzweigte Kohlenwasserstoffkette, die durch eine oder mehrere Carbonylgruppen und/oder durch ein oder mehrere Heteroatome terminiert oder unterbrochen sein kann, oder eine Kette mit einem oder mehreren aromatischen Ringen oder einem oder mehreren aromatischen oder gesättigten Heterocyclen handelt.

7. Verwendung nach Anspruch 6, **dadurch gekennzeichnet, daß** die Kohlenwasserstoffkette durch einen Hydroxy-, Alkoxy-, Amino- oder Alkylaminorest oder ein Halogen substituiert ist.

8. Verwendung nach Anspruch 6 oder 7, **dadurch gekennzeichnet, daß** es sich bei der Brückengruppe L um eine lineare oder verzweigte Kohlenwasserstoffkette mit 1 bis 40 Kohlenstoffatomen, insbesondere 1 bis 20 Kohlenstoffatomen und vorzugsweise 1 bis 8 Kohlenstoffatomen handelt.

9. Verwendung nach einem der Ansprüche 1 bis 8, **dadurch gekennzeichnet, daß** die Verbindung der Formel A-L-B unter den Verbindungen der nachstehenden Formel (I) ausgewählt ist: 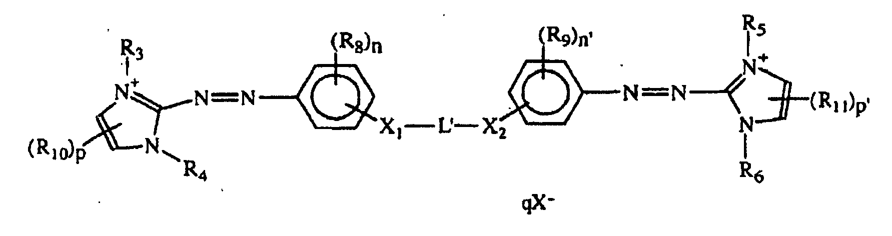 worin:
X₁ und X₂ unabhängig voneinander für einen durch einen C₁-C₈-Alkylrest, einen -O-Rest oder einen -NR₇-Rest, wobei R₇ für ein Wasserstoffatom, einen C₁-C₈-Alkylrest oder einen C₁-C₈-Hydroxyalkylrest steht, substituierten Piperazinring stehen;
R₃ bis R₆ unabhängig voneinander für einen C₁-C₈-Alkylrest oder einen C₁-C₈-Hydroxyalkylrest stehen; R₈ und R₉ für ein Wasserstoffatom, einen C₁-C₄-Alkylrest, der gegebenenfalls durch einen oder mehrere unter Hydroxy-, Amino- und C₁-C₂-(Di)alkylaminoresten ausgewählte Reste substituiert ist; einen Carboxyrest; einen C₁-C₂-Alkoxyrest; einen Aminorest; einen C₁-C₂-(Di)alkylaminorest oder einen C₂-C₄-(Poly)hydroxyalkylaminorest stehen;
R₁₀ und R₁₁ unabhängig voneinander für ein Wasserstoffatom, einen linearen oder verzweigten C₁-C₄-Alkylrest, der gegebenenfalls durch einen oder mehrere unter Hydroxy-, C₁-C₂-Alkoxy-, C₂-C₄-(Poly)hydroxyalkoxy-, Amino-, C₁-C₂-(Di)alkylamino-, Carboxy- oder Sulfonresten ausgewählte Reste substituiert ist; einen gegebenenfalls substituierten Phenylrest; einen Carboxyrest oder einen Sulfonylaminorest stehen;
L' für eine Brückengruppe steht; wobei die Gruppe X₁-L'-X₂ mindestens eine kationische Gruppe C enthält;
n und n' für eine ganze Zahl von 0 bis 4 stehen;
p und p' für eine ganze Zahl von 0 bis 2 stehen;
q für eine ganze Zahl von 3 bis 50, vorzugsweise 3 bis 10 und noch weiter bevorzugt 3 bis 5 steht;
X⁻ für ein Anion anorganischer oder organischer Herkunft steht.

10. Verwendung nach Anspruch 9, **dadurch gekennzeichnet, daß** q so gewählt ist, daß die Neutralität der Ladungen der Verbindung der Formel (I) gewährleistet ist.

11. Verwendung nach Anspruch 9, **dadurch gekennzeichnet, daß** X⁻ unter Halogenidionen, Sulfat- oder Hydrogensulfationen, Methosulfat-, Tosylat-, Carbonat-, Hydrogencarbonat-, Phosphat-, Nitrat- und Citrationen ausgewählt ist.

12. Verwendung nach einem der Ansprüche 1 bis 11, **dadurch gekennzeichnet, daß** die Verbindung der Formel A-L-B unter den Verbindungen der folgenden Formeln ausgewählt ist: 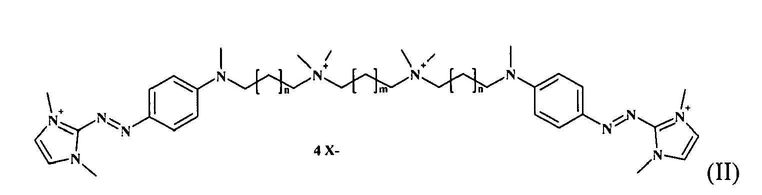 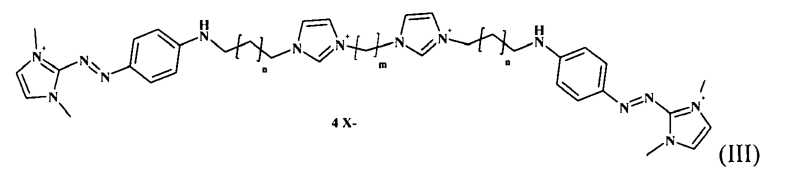 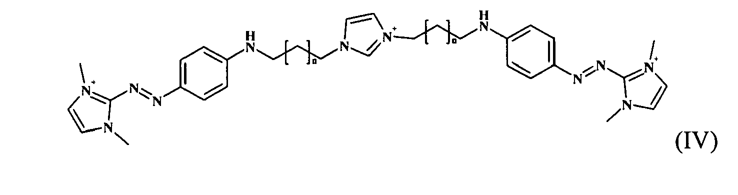 worin:
X⁻ für ein Anion anorganischer oder organischer Herkunft steht und
m und n für ganze Zahlen von 0 bis 20 und vorzugsweise 0 bis 8 stehen;
wobei m und n so beschaffen sind, daß die Brückengruppe 1 bis 40 Kohlenstoffatome, insbesondere 1 bis 20 Kohlenstoffatome und vorzugsweise 1 bis 8 Kohlenstoffatome enthält.

13. Färbezusammensetzung zum Färben von Keratinfasern, insbesondere menschlichen Keratinfasern wie den Haaren, die in einem für das Färben geeigneten Medium mindestens eine Verbindung der Formel A-L-B, worin A und B für eine Gruppe mit Färbefunktion vom Arylazoimidazolium-Typ stehen und L für eine Brückengruppe mit mindestens einer kationischen Gruppe C steht, und mindestens eine Oxidationsbase und/oder mindestens einen zusätzlichen Direktfarbstoff enthält.

14. Zusammensetzung nach Anspruch 13, **dadurch gekennzeichnet, daß** die Gruppe C unter aliphatischen oder heterocyclischen kationischen Gruppen ausgewählt ist.

15. Zusammensetzung nach Anspruch 13, **dadurch gekennzeichnet, daß** die heterocyclische kationische Gruppe unter Imidazolium-, Piperidinium-, Pyridinium-, Pyrazolium- und Triazoliumgruppen ausgewählt ist.

16. Zusammensetzung nach Anspruch 14, **dadurch gekennzeichnet, daß** es sich bei der heterocyclischen kationischen Gruppe um eine Imidazoliumgruppe handelt.

17. Zusammensetzung nach Anspruch 14, **dadurch gekennzeichnet, daß** die aliphatische kationische Gruppe unter zweiwertigen Resten des Typs -N⁺-R₁R₂-, wobei R₁ und R₂ unabhängig voneinander für einen Alkylrest mit 1 bis 10 Kohlenstoffatomen oder einen Monohydroxyalkyl- oder Polyhydroxyalkylrest mit 1 bis 10 Kohlenstoffatomen stehen, ausgewählt ist.

18. Zusammensetzung nach Anspruch 13, **dadurch gekennzeichnet, daß** es sich bei der Brückengruppe um eine gesättigte oder ungesättigte, lineare oder verzweigte Kohlenwasserstoffkette, die durch eine oder mehrere Carbonylgruppen und/oder durch ein oder mehrere Heteroatome terminiert oder unterbrochen sein kann, oder eine Kette mit einem oder mehreren aromatischen Ringen oder einem oder mehreren aromatischen oder gesättigten Heterocyclen handelt.

19. Zusammensetzung nach Anspruch 18, **dadurch gekennzeichnet, daß** die Kohlenwasserstoffkette durch einen Hydroxy-, Alkoxy-, Amino- oder Alkylaminorest oder ein Halogen substituiert ist.

20. Zusammensetzung nach Anspruch 18 oder 19, **dadurch gekennzeichnet, daß** es sich bei der Brückengruppe L um eine lineare oder verzweigte Kohlenwasserstoffkette mit 1 bis 40 Kohlenstoffatomen, insbesondere 1 bis 20 Kohlenstoffatomen und vorzugsweise 1 bis 8 Kohlenstoffatomen handelt.

21. Zusammensetzung nach einem der Ansprüche 13 bis 20, **dadurch gekennzeichnet, daß** die Verbindung der Formel A-L-B unter den Verbindungen der nachstehenden Formel (I) ausgewählt ist: 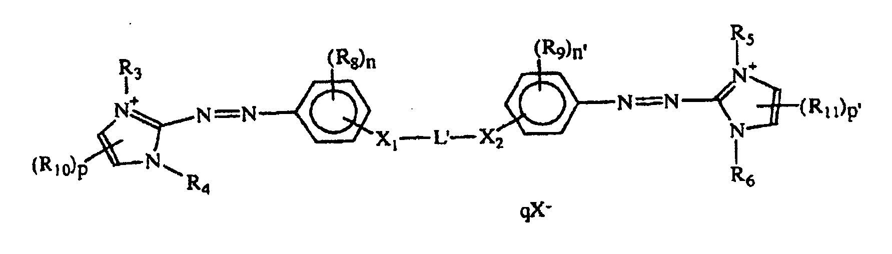 worin:
X₁ und X₂ unabhängig voneinander für einen durch einen C₁-C₈-Alkylrest, einen -O-Rest oder einen -NR₇-Rest, wobei R₇ für ein Wasserstoffatom, einen C₁-C₈-Alkylrest oder einen C₁-C₈-Hydroxyalkylrest steht, substituierten Piperazinring stehen;
R₃ bis R₆ unabhängig voneinander für einen C₁-C₈-Alkylrest oder einen C₁-C₈-Hydroxyalkylrest stehen; R₈ und R₉ für ein Wasserstoffatom, einen C₁-C₄-Alkylrest, der gegebenenfalls durch einen oder mehrere unter Hydroxy-, Amino- und C₁-C₂-(Di)alkylaminoresten ausgewählte Reste substituiert ist; einen Carboxyrest; einen C₁-C₂-Alkoxyrest; einen Aminorest; einen C₁-C₂-(Di)alkylaminorest oder einen C₂-C₄-(Poly)hydroxyalkylaminorest stehen;
R₁₀ und R₁₁ unabhängig voneinander für ein Wasserstoffatom, einen linearen oder verzweigten C₁-C₄-Alkylrest, der gegebenenfalls durch einen oder mehrere unter Hydroxy-, C₁-C₂-Alkoxy-, C₂-C₄-(Poly)hydroxyalkoxy-, Amino-, C₁-C₂-(Di)alkylamino-, Carboxy- oder Sulfonresten ausgewählte Reste substituiert ist; einen gegebenenfalls substituierten Phenylrest; einen Carboxyrest oder einen Sulfonylaminorest stehen;
L' für eine Brückengruppe steht; wobei die Gruppe X₁-L'-X₂ mindestens eine kationische Gruppe C enthält;
n und n' für eine ganze Zahl von 0 bis 4 stehen;
p und p' für eine ganze Zahl von 0 bis 2 stehen;
q für eine ganze Zahl von 3 bis 50, vorzugsweise 3 bis 10 und noch weiter bevorzugt 3 bis 5 steht;
X⁻ für ein Anion anorganischer oder organischer Herkunft steht.

22. Zusammensetzung nach Anspruch 21, **dadurch gekennzeichnet, daß** q so gewählt ist, daß die Neutralität der Ladungen der Verbindung der Formel (I) gewährleistet ist.

23. Zusammensetzung nach Anspruch 21, **dadurch gekennzeichnet, daß** X⁻ unter Halogenidionen, Sulfat- oder Hydrogensulfationen, Methosulfat-, Tosylat-, Carbonat-, Hydrogencarbonat-, Phosphat-, Nitrat- und Citrationen ausgewählt ist.

24. Zusammensetzung nach einem der Ansprüche 13 bis 23, **dadurch gekennzeichnet, daß** die Verbindung der Formel A-L-B unter den Verbindungen der folgenden Formeln ausgewählt ist: 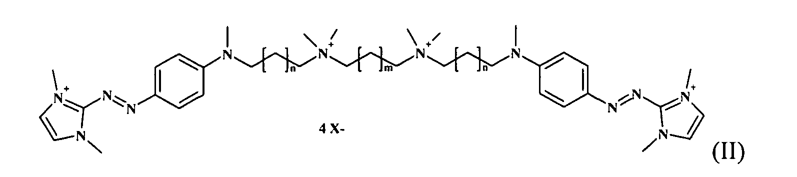 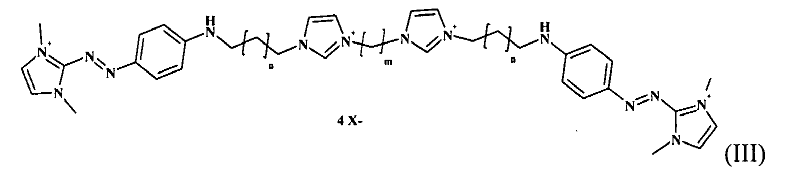 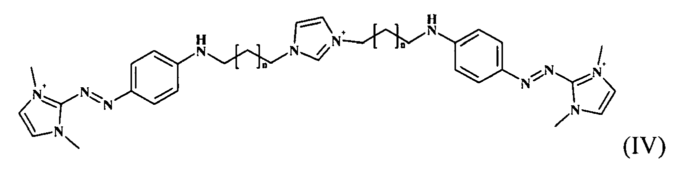 worin:
X⁻ für ein Anion anorganischer oder organischer Herkunft steht und
m und n für ganze Zahlen von 0 bis 20 und vorzugsweise 0 bis 8 stehen;
wobei m und n so beschaffen sind, daß die Brückengruppe 1 bis 40 Kohlenstoffatome, insbesondere 1 bis 20 Kohlenstoffatome und vorzugsweise 1 bis 8 Kohlenstoffatome enthält.

25. Zusammensetzung nach einem der Ansprüche 13 bis 24, **dadurch gekennzeichnet, daß** sie 0,001 bis 20 Gew.-%, vorzugsweise 0,01 bis 10 Gew.-% und noch weiter bevorzugt 0,1 bis 5 Gew.-% Direktfarbstoff bzw. Direktfarbstoffe der Formel A-L-B, bezogen auf das Gesamtgewicht der Zusammensetzung, enthält.

26. Färbezusammensetzung nach einem der Ansprüche 13 bis 25, **dadurch gekennzeichnet, daß** die Oxidationsbase unter para-Phenylendiaminen, Bis(phenyl)alkylendiaminen, para-Aminophenolen, ortho-Aminophenolen, heterocyclischen Basen und Additionssalzen davon ausgewählt ist.

27. Färbezusammensetzung nach einem der Ansprüche 13 bis 26, **dadurch gekennzeichnet, daß** die Oxidationsbase bzw. die Oxidationsbasen in einer Menge zwischen 0,001 und 20 Gew.-% und vorzugsweise zwischen 0,005 und 6 Gew.-%, bezogen auf das Gesamtgewicht der Zusammensetzung, vorliegt bzw. vorliegen.

28. Zusammensetzung nach einem der Ansprüche 13 bis 27, **dadurch gekennzeichnet, daß** der zusätzliche Direktfarbstoff unter neutralen, sauren oder kationischen Nitrobenzolfarbstoffen, neutralen, sauren oder kationischen Azodirektfarbstoffen, neutralen, sauren oder kationischen Chinon- und insbesondere Anthrachinondirektfarbstoffen, Azindirektfarbstoffen, Triarylmethandirektfarbstoffen, Indoamindirektfarbstoffen und natürlichen Direktfarbstoffen ausgewählt ist.

29. Zusammensetzung nach Anspruch 28, **dadurch gekennzeichnet, daß** der zusätzliche Direktfarbstoff 0,001 bis 20 Gew.-% und vorzugsweise 0,01 bis 10 Gew.-%, bezogen auf das Gesamtgewicht der Zusammensetzung, ausmacht.

30. Zusammensetzung nach einem der Ansprüche 13 bis 29, **dadurch gekennzeichnet, daß** sie mindestens eine unter Kupplern ausgewählte Oxidationsfarbstoffvorstufe enthält.

31. Zusammensetzung nach Anspruch 30, **dadurch gekennzeichnet, daß** der Kuppler unter meta-Phenylendiaminen, meta-Aminophenolen, meta-Diphenolen, Naphthalin-Kupplern, heterocylischen Kupplern und Additionssalzen davon ausgewählt ist.

32. Zusammensetzung nach Anspruch 30, **dadurch gekennzeichnet, daß** der Kuppler unter 1,3-Dihydroxybenzol, 1,3-Dihydroxy-2-methylbenzol, 4-Chlor-1,3-dihydroxybenzol, 2,4-Diamino-1-(β-hydroxyethyloxy)benzol, 2-Amino-4-(β-hydroxyethylamino)-1-methoxybenzol, 1,3-Diaminobenzol, 1,3-Bis(2,4-diaminophenoxy)propan, 3-Ureidoanilin, 3-Ureido-1-dimethylaminobenzol, Sesamol, 1-β-Hydroxyethylamino-3,4-methylendioxybenzol, α-Naphthol, 2-Methyl-1-naphthol, 6-Hydroxyindol, 4-Hydroxyindol, 4-Hydroxy-N-methylindol, 2-Amino-3-hydroxypyridin, 6-Hydroxybenzomorpholin, 3,5-Diamino-2,6-dimethoxypyridin, 1-N-(β-Hydroxyethyl)amino-3,4-methylendioxybenzol, 2,6-Bis(β-hydroxyethylamino)toluol und Additionssalzen davon ausgewählt ist.

33. Zusammensetzung nach einem der Ansprüche 30 bis 32, **dadurch gekennzeichnet, daß** der Kuppler bzw. die Kuppler in einer Menge zwischen 0,001 und 20 Gew.-% und vorzugsweise zwischen 0,005 und 6 Gew.-%, bezogen auf das Gesamtgewicht der Zusammensetzung, vorliegt bzw. vorliegen.

34. Zusammensetzung nach einem der Ansprüche 13 bis 33, **dadurch gekennzeichnet, daß** sie mindestens einen unter anionischen, kationischen, nichtionischen, amphoteren oder zwitterionischen Tensiden oder Mischungen davon, anionischen, kationischen, nichtionischen, amphoteren oder zwitterionischen Polymeren oder Mischungen davon, anorganischen oder organischen Verdickungsmitteln, Antioxidantien, Penetriermitteln, Sequestriermitteln, Parfümen, Puffern, Dispergiermitteln, Konditionierungsmitteln, Filmbildnern, Ceramiden, Konservierungsmitteln und Trübungsmitteln ausgewählten Hilfsstoff enthält.

35. Zusammensetzung nach einem der Ansprüche 13 bis 34, **dadurch gekennzeichnet, daß** sie mindestens ein unter Wasserstoffperoxid, Harnstoffperoxid, Alkalimetallbromaten, Persalzen, Persäuren und Oxidase-Enzymen ausgewähltes Oxidationsmittel enthält.

36. Zusammensetzung nach Anspruch 35, **dadurch gekennzeichnet, daß** es sich bei dem Oxidationsmittel um Wasserstoffperoxid handelt.

37. Verfahren zum Färben von Keratinfasern, **dadurch gekennzeichnet, daß** man eine Zusammensetzung nach einem der Ansprüche 13 bis 36 auf die Keratinfasern aufbringt und dann die Zusammensetzung auf den Fasern beläßt.

38. Verwendung einer Zusammensetzung nach einem der Ansprüche 13 bis 36 zum Färben von Keratinfasern, insbesondere menschlichen Keratinfasern wie den Haaren.

39. Verwendung einer Zusammensetzung nach einem der Ansprüche 13 bis 36 auf Keratinfasern, insbesondere menschlichen Keratinfasern wie den Haaren, zum Erhalt von Färbungen mit guter Beständigkeit gegenüber externen Faktoren und Shampoos.
